# EUROPEAN PATENT SPECIFICATION

(11) **EP 1 849 865 B1**
(45) Date of publication and mention of the grant of the patent: **22.05.2013**
(21) Application number: 06712960.1
(22) Date of filing: 27.01.2006
(51) Int. Cl.: C12N 15/11, C12N 15/09, A61K 48/00, A61P 31/04, A61P 31/10, A61P 31/12, A61P 35/00

(54) **RNAi MEDICINE HAVING NO ADVERSE EFFECTS**
RNAi-MEDIZIN OHNE NACHTEILIGE EFFEKTE
AGENT THÉRAPEUTIQUE À BASE D'ARNi NE PRÉSENTANT AUCUN EFFET SECONDAIRE INDÉSIRABLE

(30) Priority: 28.01.2005 JP 2005021960
(43) Date of publication of application: 31.10.2007
(73) Proprietor: Haplopharma Inc., Okinawa 904-2234 (JP)
(72) Inventor: TAKAKU, Hiroshi, Funabashi-shi, Chiba 2740063 (JP); KUROSAKI, Naoko, Tokyo 1360072 (JP); YAMAGUCHI, Kazuya, Sodegaura-shi, Chiba 2990245 (JP); GONDAI, Takuma, Kuki-shi, Saitama 346-0005 (JP)
(74) Representative: Denison, Christopher Marcus
(86) International application number: PCT/JP2006/301817
(87) International publication number: WO 2006/080564

(56) References cited:
- KATOH TAKAYUKI ET AL: "Simple and rapid synthesis of siRNA derived from in vitro transcribed shRNA." NUCLEIC ACIDS RESEARCH. SUPPLEMENT (2001) 2003, no. 3, 2003, pages 249-250, XP002537121
- KIM DONG-HO ET AL: "Interferon induction by siRNAs and ssRNAs synthesized by phage polymerase" NATURE BIOTECHNOLOGY, NATURE PUBLISHING GROUP, NEW YORK, NY, US, vol. 22, no. 3, 1 March 2004 (2004-03-01), pages 321-325, XP002428031 ISSN: 1087-0156
- SLEDZ C A ET AL: "ACTIVATION OF THE INTERFERON SYSTEM BY SHORT-INTERFERING RNAS" NATURE CELL BIOLOGY, NATURE PUBLISHING GROUP, GB, vol. 5, no. 9, 1 September 2003 (2003-09-01), pages 834-839, XP009043516 ISSN: 1465-7392
- PERSENGIEV STEPHAN P ET AL: "Nonspecific, concentration-dependent stimulation and repression of mammalian gene expression by small interfering RNAs (siRNAs)" RNA, COLD SPRING HARBOR LABORATORY PRESS, WOODBURY, NY, US, vol. 10, no. 1, 1 January 2004 (2004-01-01), pages 12-18, XP002369414 ISSN: 1355-8382
- KARIKO KATALIN ET AL: "Small interfering RNAs mediate sequence-independent gene suppression and induce immune activation by signaling through toll-like receptor 3" JOURNAL OF IMMUNOLOGY, AMERICAN ASSOCIATION OF IMMUNOLOGISTS, US, vol. 172, no. 11, 1 June 2004 (2004-06-01), pages 6545-6549, XP002482882 ISSN: 0022-1767
- BRIDGE A J ET AL: "Induction of an interferon response by RNAi vectors in mammalian cells" NATURE GENETICS, NATURE PUBLISHING GROUP, NEW YORK, US, vol. 34, no. 3, 1 July 2003 (2003-07-01), pages 263-264, XP002251051 ISSN: 1061-4036
- PEBERNARD S ET AL: "Determinants of interferon-stimulated gene induction by RNAi vectors" DIFFERENTIATION, SPRINGER VERLAG, DE, vol. 72, 1 March 2004 (2004-03-01), pages 103-111, XP002383799 ISSN: 0301-4681
- GONDAI TAKUMA ET AL: "Lack of interferon (IFN) response to T7 Transcribed pppG (n)(n = 2,3)-shRNA." NUCLEOSIDES, NUCLEOTIDES & NUCLEIC ACIDS 2007, vol. 26, no. 6-7, 2007, pages 805-808, XP002537122 ISSN: 1525-7770
- GONDAI TAKUMA ET AL: "Short-hairpin RNAs synthesized by T7 phage polymerase do not induce interferon." NUCLEIC ACIDS RESEARCH FEB 2008, vol. 36, no. 3, February 2008 (2008-02), page e18, XP002537123 ISSN: 1362-4962
- SONG J. ET AL.: 'Poly(U) and polyadenylation termination signals are interchangeable for terminating signals are interchangeable for terminating the expression of shRNA from a pol II promoter' BIOCHEM. BIOPHYS. RES. COMMUN. vol. 323, no. 2, 2004, pages 573 - 578, XP002380611
- HANYU Y. ET AL.: 'HIV-1 vif shRNA Oyobi hu-APOBEC3G no Kyohatsugen ni yoru HIV-1 Kansen Sogai' DAI 27 KAI ANNUAL MEETING OF THE MOLECULAR BIOLOGY SCIENTY OF JAPAN PROGRAM KOEN YOSHISHU vol. 27TH, 2004, page 878, 3PA-336, XP003004069
- TAKAKU H. ET AL.: 'Specific silencing of HIV-1 env gene using small interfering RNAs in mammalian cells' ANTIVIRAL RES. vol. 57, no. 3, 2003, page A44, 15, XP003004070
- ARRIGHI J.F. ET AL.: 'Lentivirus-mediated RNA interference of DC-SIGN expression inhibits human immunodeficiency virus transmission from dendritic cells to T cells' J. VIROL. vol. 78, no. 20, 2004, pages 10848 - 10855, XP003004071

## Description

### Technical Field

The present invention relates to double-strand RNA (dsRNA) that causes inhibition (silencing) of gene expression in a sequence-specific manner without interferon induction.

### Background Art

RNA interference (RNAi) is referred to as a phenomenon in which mRNA is cleaved by double-strand RNA (dsRNA) in a sequence-specific manner, resulting in inhibition of gene expression. RNAi has been reported to be a nucleic-acid-level protection system common in organisms (see Waterhouse, P.M. et al., Nature, 411: 834-843, 2001). Upon RNAi, a dicer functions to process dsRNA so that siRNA (short interfering RNA) is formed. Then, siRNA serving as a guide RNA recognizes a target sequence so as to cleave target mRNA, resulting in inhibition of gene expression.

RNAi methods have been widely examined for the purposes of gene function analysis based on regulation of gene expression, elucidation of a mechanism for regulating gene expression, use of RNAi for gene therapies, and the like.

Regarding RNAi, induction of interferon (α, β) expression and cytotoxicity caused by dsRNA have been reported (see K. Kariko et al., Cells Tissues Organs 2004, 177, 132-138; S. Pebernard et al., Differentiation (2004) 72, 103-111; C. A. Sledz et al., Nature Cell Biology, advance online publication, 24 August 2003; D. H. Kim et al., Nature Biotechnology volume 22, Number 3, 321-325, 2004; A. J. Bridge et al., Nature Genetics, volume 34, number 3, 263-264, 2004; S. P. PERSENGIEV et al., RNA (2004), 10, 12-18; and K. Kariko et al., J. of Immunol., 2004, 172, 6545-6549, 2004). For instance, it has been reported that, when dsRNA is recognized via a Toll-like receptor 3 (TLR3), the expression of interferon type I is induced by TLR3 in addition to induction of sequence-specific gene expression, resulting in induction of 2'-5'-oligoadenylate synthetase that degrades RNA in a sequence-nonspecific manner. Also, the dsRNA-dependent protein kinase (PKR) has been reported to be activated by siRNA so as to upregulate IFN-β expression.

As described above, with conventional RNAi methods, induction of the interferon expression caused by dsRNA results in induction of an interferon reaction or cytotoxicity, which has been problematic. In a case in which dsRNA is used as an experimental reagent, nonspecific expression inhibition precludes accurate evaluation of effects of dsRNA used, which is problematic. Further, in a case in which dsRNA is used as a medicine, nonspecific expression inhibition or cytotoxicity is induced so that a test subject experiences adverse effects, which is also problematic.

### Disclosure of the Invention

It is an objective of the present invention to provide an RNAi reagent and an RNAi medicine that have no adverse effects, whereby interferon expression and cytotoxicity are induced.

In view of the above problems, the inventors of the present invention have conducted intensive studies of the development of dsRNA that can cause RNAi without inducing interferon expression. As a result, they have found that shRNA does not induce interferon expression and non-specific RNA degradation or cytotoxicity when shRNA having a loop structure is allowed to have an overhang comprising one or a plurality of G(s) at the 5' end of a sense strand thereof. This has led to the completion of the present invention.

Specifically, embodiments of the present invention are as follows.
[1] An shRNA comprising a sense strand comprising a sequence homologous to a target sequence of target mRNA and an antisense strand comprising a sequence complementary to the sequence of the sense strand and having an overhang consisting of two or three G base(s) at the 5' end of the sense strand and triphosphoric acid is ligated to the 5' end of the overhang such shRNA cleaving target mRNA in a sequence-specific manner without causing interferon and/or cytotoxicity induction.
[2] The shRNA according to any one of [1], wherein the sense strand and the antisense strand have base lengths of 15 to 50.
[3] A vector containing template DNA of the shRNA according to any one of [1] to [2] and expressing the shRNA, wherein the vector comprises a T7 promoter.
[4] A gene expression inhibitor that inhibits *in vitro* the expression of a target gene or a noncoding region comprising a target sequence in a cell without interferon and/or cytotoxicity induction, such inhibitor comprising the shRNA according to any one of [1] to [2].
[5] A method of inhibiting *in vitro* the expression of a target gene or a noncoding region comprising a target sequence in a cell without interferon and/or cytotoxicity induction, comprising introducing the shRNA according to any one of [1] to [2] into a cell.
[6] A pharmaceutical composition that does not cause interferon and/or cytotoxicity induction so as to prevent and/or treat a disease involving a gene or a noncoding region comprising a target sequence, such pharmaceutical composition comprising, as an active ingredient, the shRNA according to any one of [1] to [2].
[7] The pharmaceutical composition according to [6], wherein the target sequence is a gene sequence or a noncoding region of a virus and the disease is a viral infectious disease.
[8] The pharmaceutical composition according to [7], wherein the target sequence is a gene sequence or a noncoding region of HIV.

### Brief Description of the Drawings

Fig. 1 shows the base sequence of pppGG-shRNA.
Fig. 2 shows the structure of template DNA.
Fig. 3A and 3B show pictures indicating results of examining IFN-β induction and cytopathic effects (CPEs) caused by pppGG-shDIS. Fig. 3A shows results of Western blotting for IFN-β detection and fig. 3B shows CPEs arising due to pppGG-shDIS introduction.
Fig. 4 shows the base sequence of ppp-shDIS.
Fig. 5A to 5C show pictures indicating the influence of the shRNA 5' overhang upon IFN induction. Fig. 5A shows CPE results, fig. 5B shows results of Western blotting, and fig. 5C shows results of ELISA.
Fig. 6A and 6B show a target sequence of shLuc2 and the structure of shLuc2.

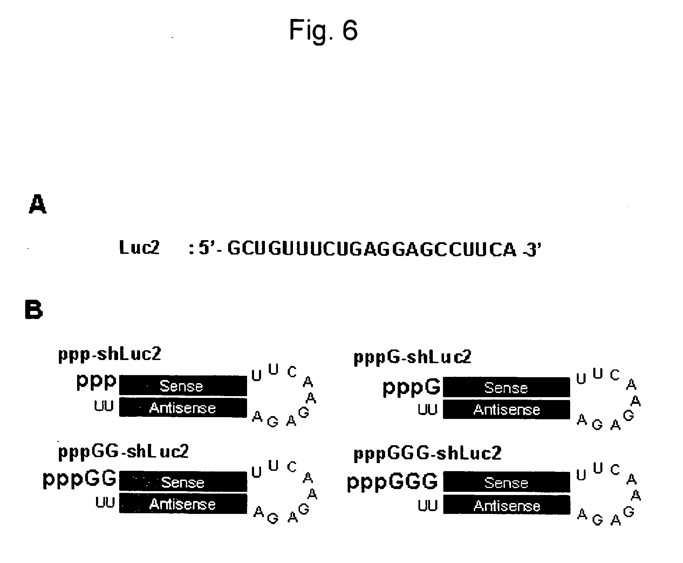

   Fig. 6A shows a target sequence of shLuc2 and fig. 6B shows the structure of shLuc2.

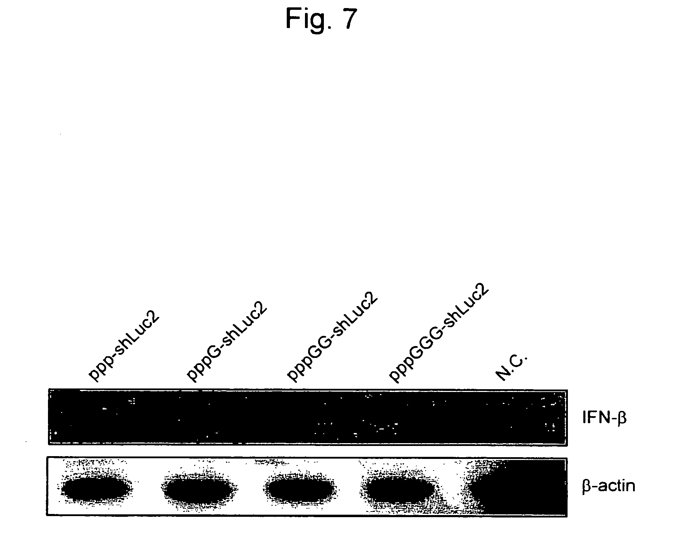

Fig. 7 shows pictures of IFN-β induction dependent on the number of bases in the 5' overhang.

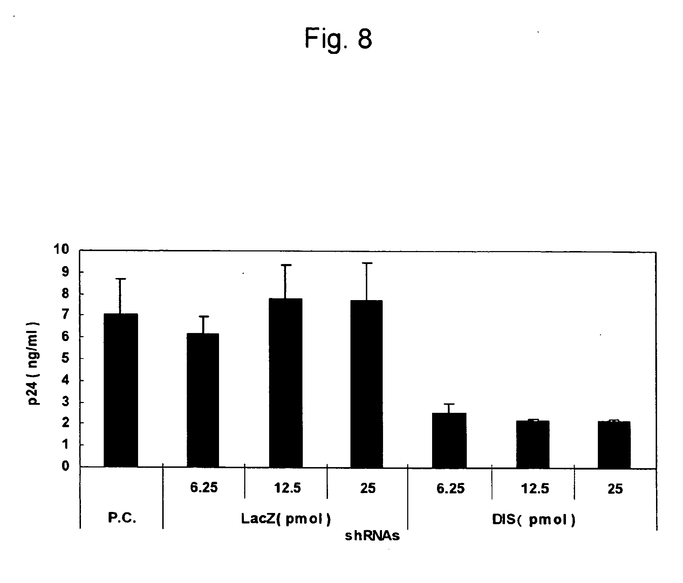

Fig. 8 shows anti-HIV effects of ppp-GGshDIS.

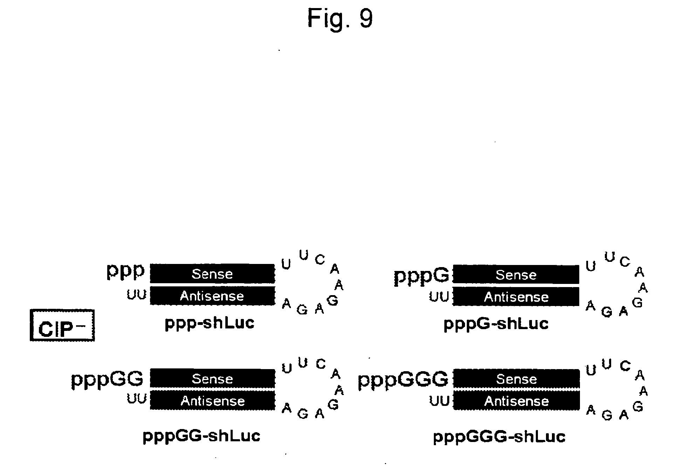

Fig. 9 shows the structure of ppp-shLuc6 targeting firefly luciferase.

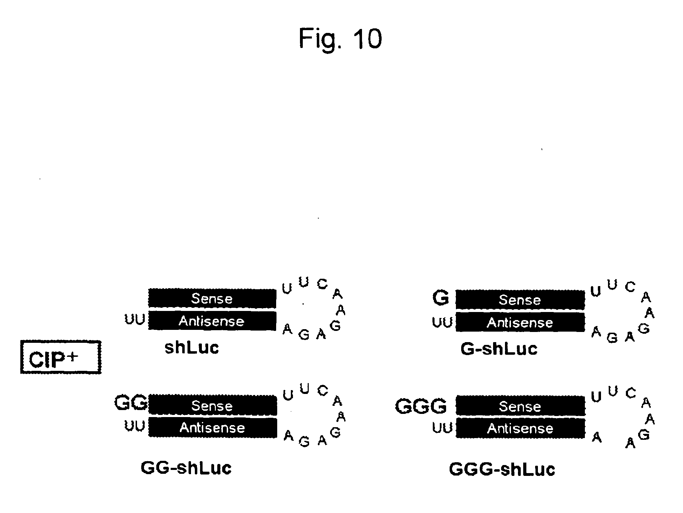

Fig. 10 shows the structure of dephosphorylated shLuc6.

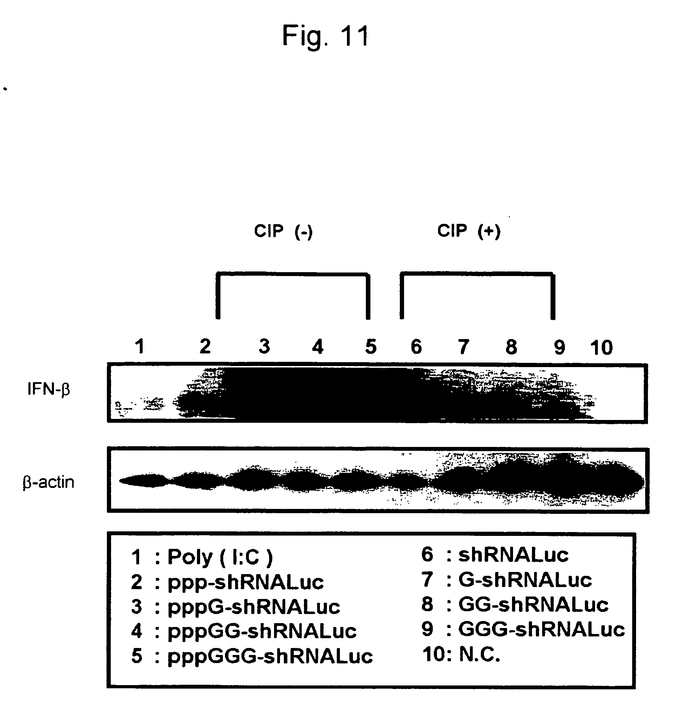

Fig. 11 shows pictures indicating results of Western blotting for a test to confirm IFN β production.

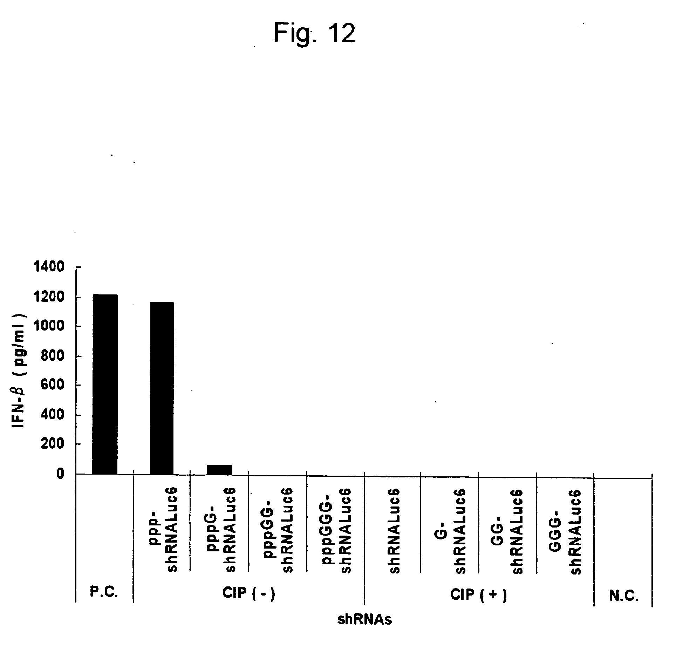

Fig. 12 shows results of ELISA for a test to confirm IFN β production.

### Best Mode for Carrying Out the Invention

Hereafter, the present invention will be described in greater detail.

In the present invention, short hairpin RNA (shRNA) is used, such shRNA comprising a double-strand portion and having a stem-loop structure in which a sense strand is ligated to an antisense strand via a loop sequence. Such double-strand structure is formed with a self-complementary RNA strand in which a single RNA strand contains a sense strand and an antisense strand that serve as reverse sequences to each other. Short hairpin RNA is subjected to intracellular or *in vivo* processing, resulting in production of siRNA. The shRNA of the present invention has an overhang at the 5' end of a sense strand thereof. Such overhang consists of two or three, guanine(s) (G(s)). With the presence of an excessive number of G, the overhang portion forms a quadruple-strand structure so that, in some cases, such overhang portion is not recognized by a dicer. The appropriate number of G can readily be determined by administering the shRNA , to which an overhang comprising G(s) has been added, to cells and measuring the inhibitory efficacy of gene expression and the degree of interferon and/or cytotoxicity induction. In addition, triphosphoric acid (ppp) is ligated to the 5' side of an overhang comprising G(s). Further, the 3' end of an antisense strand may have an overhang. Such overhang is not limited in terms of type or number of bases. For instance, such overhang has a sequence comprising 1 to 5, preferably 1 to 3, and further preferably 1 or 2 base(s). An example of such sequence is represented as "UU." In addition, according to the present invention, the term "overhang" denotes base(s) added to the end of one strand of shRNA, such overhang not comprising base(s) capable of complementarily binding to base(s) located at the corresponding site of the other strand.

The aforementioned double-strand portion has a structure in which an RNA strand (sense strand) having a sequence capable of hybridizing to a specific target sequence contained in a sequence of a target gene or a noncoding region to be knocked down by RNAi is complementarily bound to an RNA strand (antisense strand) that is complementary to the sequence bound thereto.

In the shRNA of the present invention, the 3' end of a sense strand is ligated to the 5' end of an antisense strand via a loop (hairpin loop sequence). Examples of a hairpin loop sequence include, but are not limited to, a sequence comprising 5 to 12 bases starting with "UU," such as "UUCAAGAGA" (SEQ ID NO: 1). Other examples of such loop sequence that can be used include loops comprising sequences described in, for example, Lee NS. et al. (2002) Nat. Biotech. 20, 500-505; Paddison PJ. et al. (2002) Genes and Dev. 16, 948-958; Sui G. et al. (2002) Proc. Natl. Acad. Sci. USA 99, 5515-5520; Paul CP. et al. (2002) Nat. Biotech. 20, 505-508; and Kawasaki H. et al. (2003) Nucleic Acids Res. 31, 700-707.

The shRNA of the present invention comprises an overhang at the 5' end of a sense strand, a sense strand, a loop sequence, and an antisense strand and has a structure in which the sense strand is complementarily bound to the antisense strand. Further, as described above, the shRNA of the present invention may comprise an overhang comprising base(s) at the 3' end of an antisense strand. Neither the portion having an overhang comprising base(s) nor the loop portion has a double-strand structure. However, according to the present invention, RNA contains a double-strand portion comprising a sense strand and an antisense strand so that such RNA is referred to as double-strand RNA in some cases.

The term "target gene" used herein includes genes derived from organisms to which the shRNA of the present invention is administered and genes of viruses, bacteria, fungi, protozoans, and the like that exist in organisms by infecting such organisms. Examples of genes derived from organisms include oncogenes, antioncogenes, genes involved in development, genes encoding enzymes, and disease-related genes that cause diseases. In addition, examples of viruses and bacteria include HIV, HCV, HBV, HTLV-1, HTLV-2, influenza virus, SARS coronavirus, rotavirus, norovirus, enterohemorrhagic *Escherichia coli, Mycobacterium* tuberculosis, methicillin-resistant *Staphylococcus aureus* (MRSA), *Pseudomonas aeruginosa,* enterococcus, *Candida,* hemolytic streptococcus, *Helicobacter pylori,* syphilis spirochete, and *Chlamydia trachomatis.* In the case of the shRNA of the present invention, the selectable number of bases of a specific target sequence of a target gene or a noncoding region may be, but is not limited to, between 15 and 500. Preferably, such number of bases is 15 to 50, 15 to 45, 15 to 40, 15 to 35, or 15 to 30, further preferably 20 to 35, further preferably 19 to 30, and particularly preferably 19 to 29 or 28. It has been reported that an interferon reaction is likely to be induced in proportion to the length of shRNA (JP Patent Publication (Kokai) No. 2003-219893 A). However, in the case of the shRNA of the present invention, the addition of an overhang comprising G(s) to the 5' end of a sense strand results in inhibition of an interferon reaction. Thus, even in the case of a target sequence having a long length, an interferon reaction is not induced. A target sequence of a target gene or a noncoding region may be adequately selected as a sequence, for example, in which sizable effects of inhibiting the target gene expression are observed. Preferably, the shRNA of the present invention is identical to a target sequence. However, the shRNA may be a sequence substantially identical (namely, homologous) to a target sequence. That is, as long as a sense strand sequence of the shRNA of the present invention hybridizes to a target sequence, these sequences may differ from each other by 1 or more (namely, 1 to 10, preferably 1 to 5, and further preferably 1 to 3, 2, or 1) mismatched base(s). In such case, hybridization takes place under *in vivo* conditions when the shRNA of the present invention is administered *in vivo* so as to be used as a medicine or under moderately or highly stringent conditions when the shRNA of the present invention is used *in vitro* as a reagent. For instance, hybridization takes place under conditions involving the presence of 400 mM NaCl, 40 mM PIPES (pH 6.4), and 1 mM EDTA at 50°C to 70°C for 12 to 16 hours. In addition, a sense strand sequence of the shRNA of the present invention has 90% or more, preferably 95% or more, and further preferably 95%, 96%, 97%, 98%, or 99% or more sequence homology to a target sequence.

The shRNA of the present invention can be synthesized *in vitro* via chemical synthesis or in a transcription system with the use of a promoter and an RNA polymerase. Upon chemical synthesis, a self-complementary single-strand RNA having sequences complementary to each other as reverse sequences is synthesized such that self-complementary portions of the single-strand RNA are allowed to be bound to each other. In addition, upon synthesis with the use of a promoter and an RNA polymerase, template DNA having a structure in which a sense strand and an antisense strand are ligated to each other via a loop is synthesized downstream of a single promoter so that RNA transcription may be carried out by an RNA polymerase. In order to add an overhang sequence comprising G(s) to the 5' end of a sense strand of shRNA, a sequence comprising G(s) may be added to the end of a promoter. In such case, a DNA sequence is allowed to comprise a terminator or the like according to need. As a terminator sequence, a sequence represented as "TTTTTT" (SEQ ID NO: 2) may be used, for example. *Upon in vitro* production, promoters such as a T3 promoter and a T7 promoter are used. When the double-strand RNA of the present invention is synthesized *in vivo* by introducing template DNA of the double-strand RNA into a vector followed by *in vivo* administration of the vector, PolIII promoters such as a U6 promoter and an H1 promoter are used, for example. When a vector is used, examples of a vector that can be used include plasmid vectors and viral vectors. Examples of plasmid vectors that may be used include a pBAsi vector and a pSUPER vector. Examples of viral vectors that may be used include an adenovirus vector, a lentivirus vector, and a retrovirus vector. In addition, in a case in which a T7 promoter or the like is used for synthesizing the shRNA of the present invention, a T7 promoter is activated due to the presence of a sequence comprising G(s) so that the production efficiency of shRNA is advantageously enhanced.

The shRNA of the present invention may be introduced into a test subject that is a target for the expression inhibition. Examples of such test subject include, but are not limited to, cells, tissues, and individuals. Examples of an individual used as a test subject include animals in which an interferon reaction can be induced. The shRNA of the present invention can be applied as a medicine for prevention or treatment of a disease to animals. However, methods for treatment of the human or animal body by therapy are not claimed. Further, the shRNA of the present invention can be used as a research reagent. In such case, the shRNA of the present invention can be used as a gene expression inhibitor, an RNAi reagent, or the like. When the shRNA of the present invention is used as a reagent, it is possible to *inhibit in vitro* the expression of a target gene containing a target sequence in a cell without interferon and/or cytotoxicity induction by introducing shRNA into the cell. The term *"in vitro"* according to the present invention includes a condition in which the shRNA of the present invention is administered *ex vivo*. For instance, also in a case in which shRNA is introduced into a cell or tissue placed in a test tube, it can be said that shRNA is introduced *in vitro.*

A disease to which the shRNA of the present invention is applied for the purpose of treating or preventing such disease is a disease involving a gene that is a target of the expression inhibition caused by shRNA. Specific examples of such disease include: cancers involving oncogenes such as the APC gene (large intestine cancer) and the BRCA1/BRCA2 gene (breast cancer); and neurodegenerative diseases involving transcriptional abnormality of a variety of genes such as the PS1 gene (Alzheimer's disease), the LPL gene (hyperlipidemia), and the INS/INSR gene (diabetes). Examples of cancers that can be treated with the shRNA of the present invention include esophageal cancer, gastric cancer, large intestine cancer, hepatocellular carcinoma, pancreatic cancer, cholangiocarcinoma, breast cancer, lung cancer, lung adenocarcinoma, kidney cancer, urinary bladder cancer, prostate cancer, corpus uteri cancer, cervical cancer, nasopharyngeal cancer, thyroid cancer, ovarian cancer, skin cancer, leukemia, myeloma, lymphoma, and lymphosarcoma. Also, in the cases of these cancers, the expression of the relevant oncogene may be inhibited. In addition, in the cases of infectious diseases caused by microorganisms such as viruses and bacteria, the expression of the relevant viral gene or bacterial gene may be inhibited. Examples of such viruses and bacteria include HIV, HCV, HBV, HTLV-1, HTLV-2, influenza virus, SARS coronavirus, rotavirus, norovirus, enterohemorrhagic *Escherichia coli, Mycobacterium* tuberculosis, methicillin-resistant *Staphylococcus aureus* (MRSA), *Pseudomonas aeruginosa,* enterococcus, *Candida,* hemolytic streptococcus, *Helicobacter pylori,* syphilis spirochete, and *Chlamydia trachomatis.* The entire or a partial genome sequence of such virus or bacterium and the genetic information are known, making it possible to select a target sequence in accordance with the genome and the genetic information.

In a case in which a test subject is a cell or tissue, the shRNA of the present invention can be introduced by a method wherein the shRNA of the present invention is simultaneously cultured together with such cell or tissue. Other examples of a method of introducing the shRNA of the present invention include a method using calcium ions, the electroporation method, the spheroplast method, the lithium acetate method, the calcium phosphate method, the lipofection method, and the microinjection method. In a case in which a test subject is an animal individual, the shRNA of the present invention can be administered via oral routes, via intravenous, intramuscular, subcutaneous, or intraperitoneal injections, or via nonenteral routes. Further, when the shRNA of the present invention is administered to a specific site, the shRNA of the present invention may be delivered to such specific site with the use of a drug delivery system. Various types of drug delivery systems have been known to the public. Depending on the site to which the shRNA of the present invention is administered, it is possible to select an adequate method. Examples of a method using a drug delivery system include known methods using carriers such as liposome, emulsion, and polylactic acid. Upon administration, it is preferable to mix the shRNA of the present invention with a pharmaceutically acceptable diluent or carrier. Examples of an appropriate carrier include, but are not limited to, physiological saline, phosphate-buffered physiological saline, phosphate buffered physiological saline glucose liquid, and buffered physiological saline. The amount of shRNA to be introduced can be adequately determined depending on, for example, the type of a disease to be prevented or treated, the severity of the disease, and the age and the weight of a test subject. The preferred amount of shRNA is an amount at which at least one copy of shRNA is introduced per cell in a lesion.

According to the present invention, the state in which inhibition (silencing) of the expression of a target gene or a noncoding region is caused by RNA interference includes not only a state in which the expression is inhibited by 100% but also a state in which the expression is inhibited by 75% or more, 50% or more, or 20% or more with respect to a case in which the shRNA of the present invention is not introduced when the expression of a gene is judged based on the expression level of a protein or mRNA of the gene or a noncoding region. The degree of expression inhibition may be determined by comparing the amount of mRNA or a protein of a gene or a noncoding region produced before shRNA introduction with the same after shRNA introduction. The amount of mRNA produced can be measured by Northern hybridization, *RT-PCR*, *in situ* hybridization, or the like. The amount of protein produced can be measured by Western blotting, ELISA, a measurement method using an antibody-bound protein chip, a method for measuring protein activity, or the like. In addition, the shRNA of the present invention is characterized in that it does not induce an interferon reaction when introduced into a cell or an individual. A state in which an interferon is not induced is a state in which the expression of interferon α or β is not induced. In such state, not only synthesis of an interferon but also activation of a pathway involving an interferon is not induced. Also, such state includes not only a state in which the interferon expression is inhibited by 100% but also a state in which the interferon expression is inhibited by 75% or more, 50% or more, 20% or more, 10% or more. The presence or absence of an interferon reaction can be determined by measuring the amount of an interferon or mRNA of such interferon produced. Such measurement may be carried out by Northern hybridization, *RT-PCR*, *in situ* hybridization, Western blotting, ELISA, a measurement method using an antibody-bound protein chip, or a method for measuring protein activity as described above. Further, the shRNA of the present invention is characterized in that it does not induce cytotoxicity in a cell or individual even when introduced into the cell or individual. In general, upon administration of a double-strand RNA, activation of dsRNA-dependent protein kinase (PKR) results in induction of cytotoxicity. However, the shRNA of the present invention does not activate PKR and does not induce cytotoxicity. Herein, the term "cytotoxicity" indicates a state in which a cell does not exert its normal functions or a cell experiences disorders to an extent that results in growth inhibition. Cytotoxicity includes cell death such as apoptosis or necrosis. According to the present invention, a state in which no cytotoxicity is induced includes not only a state in which no cytotoxicity is induced but also a state in which the number of cells that experience cytotoxicity is 75% or less, 50% or less, 20% or less, or 10% or less than the number of such cells in a case in which double-strand RNA in which a sequence comprising G(s) has not been introduced into the 5' end of a sense strand is introduced. It is possible to confirm whether or not cytotoxicity is induced by observing cells and examining whether or not cytopathic effects (CPEs) are observed. Also, it is possible to judge whether or not cytotoxicity is induced by measuring metabolic activity of cells or performing a dye (e.g. trypan blue) exclusion assay. The shRNA of the present invention does not induce interferon and/or cytotoxicity. As described above, according to the present invention, the expression "without interferon and/or cytotoxicity induction" indicates not only a state in which such induction is completely inhibited but also a state in which such induction is attenuated. In addition, the degree of inhibition of interferon and/or cytotoxicity induction differs depending on experimental systems or test subjects in some cases. However, as long as shRNA does not induce interferon and/or cytotoxicity in at least one such system or test subject, such shRNA is included in the shRNA of the present invention that does not induce interferon and/or cytotoxicity.

Further, disclosed herein is a method of administering the shRNA of the present invention to an organism so as to inhibit the expression of a target gene or a noncoding region containing a target sequence in such organism without induction of interferon expression; and a method of administering the shRNA of the present invention to an animal so as to prevent and/or treat a disease involving a target gene or a noncoding region containing a target sequence without induction of interferon expression. Furthermore, the present invention encompasses the use of the shRNA of the present invention for producing a medicine for prevention and/or treatment of a disease involving a target gene containing a target sequence of the shRNA of the present invention without induction of interferon expression.

The present invention is hereafter described in greater detail with reference to the following examples, although the technical scope of the present invention is not limited thereto.

### Example 1 Synthesis of interferon-noninducible shRNA

### (1) Synthesis of shRNA (pppGG-shDIS)

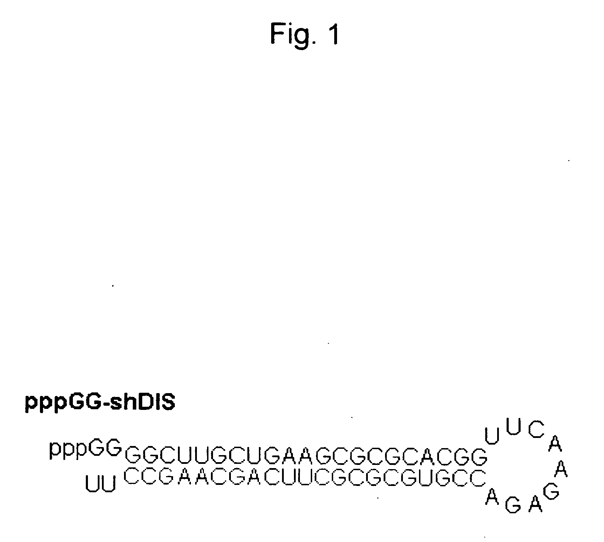

shRNA (shDIS) was synthesized based on the base sequence of a DIS (dimerization initiation site) of HIV-1 (human immunodeficiency virus type 1). Fig. 1 shows the sequence of shRNA. As shown in fig. 1, shRNA (pppGG-shDIS) corresponding to HIV-1 DIS comprises triphosphoric acid, GG, a sense strand (5'-GGCUUGCUGAAGCGCGCACGG-3' (SEQ ID NO: 3)), a loop sequence (5'-UUCAAGAGA-3' (SEQ ID NO: 1)), an antisense strand, and UU in that order from the 5' end thereof.

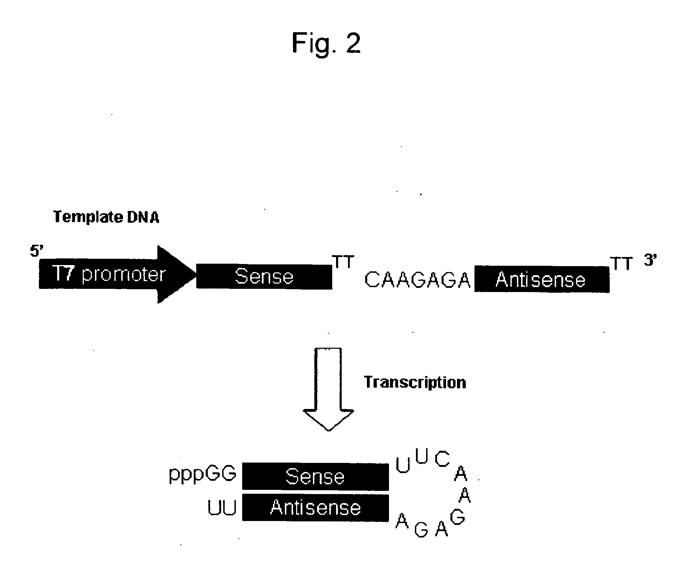

shRNA was synthesized with T7 RNA polymerase (AmpliScribe™ T7- Flash™ Transcription Kits, EPICENTRE, Cat. No. ASF3507) by a method using a double-strand DNA having a short hairpin RNA (shRNA) sequence to which a T7 promoter (5'-TAATACGACTCACTATAGG -3' (SEQ ID NO: 4)) had been added (fig. 2). As shown in fig. 2, such DNA comprises a T7 promoter, a sense strand, a loop sequence, an antisense strand, and UU in that order form the 5' end thereof. When a T7RNA polymerase is allowed to act on the above DNA, shRNA shown in fig. 1 is synthesized (SEQ ID NO: 5). In fig. 2, a sense strand, to which an antisense strand complementary thereto has been bound, is exclusively described. In the case of the sequence represented by SEQ ID NO: 5, HIV-1 DIS was used as a target sequence. In addition, a sense strand may comprise any target sequence.

### (2) Evaluation of the interferon β-inducing capacity of pppGG-shDIS

pppGG-shDIS was introduced into HeLa CD4⁺ cells, followed by examination of interferon β (IFN β)-inducing capacity.

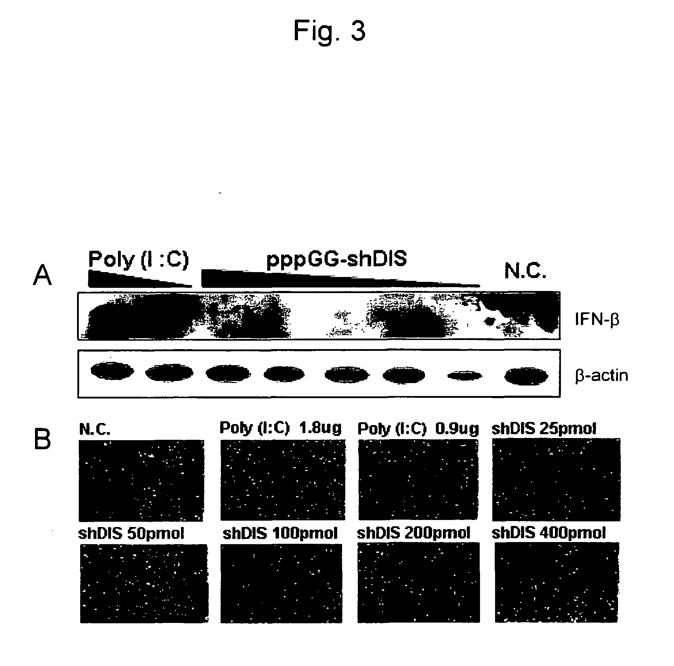

First, HeLa CD4⁺ cells were seeded on a 6-well plate at 1.4 x 10⁵ cells/well, followed by culture in RPMI-1640 (containing 10% FBS, 100 U/ml penicillin, and 100 µg/ml streptomycin) for a day (37°C, 5% CO₂ atmosphere). After culture, the medium was completely removed therefrom and 800 µl of RPMI-1640 (containing 100 U/ml penicillin and 100 µg/ml streptomycin) was freshly added dropwise thereto. DMRIE-C (Invitrogen) and Opti-MEM (Invitrogen) were added to pppGG-shDIS (400, 200, 100, 50, or 25 pmol) (shRNA : reagent = 1 µg : 3 µL) and each resultant was adjusted to 200 µL, followed by incubation at room temperature for 20 minutes. The thus incubated pppGG-shDIS-reagent complex was added dropwise to HeLa CD4⁺ cells, followed by culture for 1.5 hours. Thus, pppGG-shDIS was introduced into the cells. At the same time, Poly (I:C) (1.8 or 0.9 µg) serving as a control for IFN β induction was introduced into the cells in the manner described above. After culture, the HeLa CD4⁺ cells were washed with RPMI-1640 (containing 100 U/ml penicillin and 100 µg/ml streptomycin), and 1 mL of RPMI-1640 (containing 10% FBS, 100 U/ml penicillin, and 100 µg/ml streptomycin) was added dropwise thereto, followed by culture for 12 hours. After culture, cells were recovered, followed by analysis of intracellular IFN β expression by the Western blotting method (fig. 3A). In such case, Poly (I:C) was used as a control. In the figure, a symbol "N.C." represents a sample into which nothing had been introduced.

In addition, pppGG-shDIS was introduced in the manner described above, followed by culture for 48 hours. Cytopathic effects (CPEs) were microscopically observed (fig. 3B).

As shown in fig. 3A, in the case of Poly (I:C) used as a control, IFN-β induction was observed. On the other hand, in the case of pppGG-shDIS introduced at any of the above amounts, IFN-β induction was not observed. In addition, CPEs were confirmed in the systems to which pppGG-shDIS was separately introduced at 200 and 400 pmol. Based on the above results, it was found that pppGG-shDIS does not induce IFN. Further, it was suggested that PKR/2-50AS would be possibly induced.

### (3) Synthesis of shRNA (ppp-shDIS)

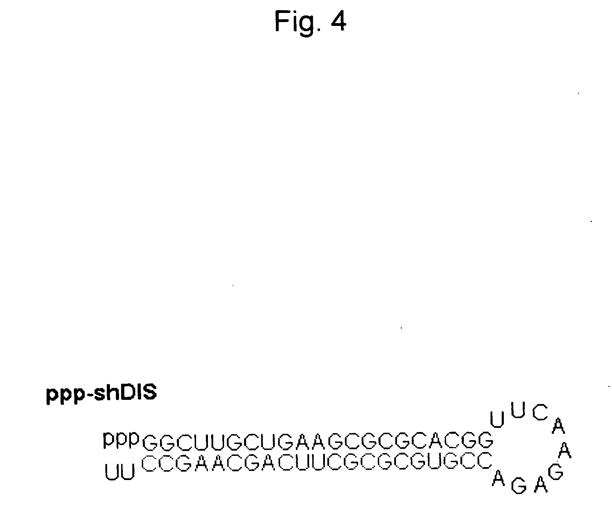

In the case of pppGG-shDIS, IFN β induction was not observed. Thus, a different type of shRNA (ppp-shDIS) was synthesized (fig. 4). Such ppp-shDIS lacks GG, which is contained in the 5' overhang of pppGG-shDIS. These two types of shRNAs were examined in terms of IFN β-inducing capacity.

ppp-shDIS was synthesized by a method wherein the base sequence of a T7 promoter (5'-TAATACGACTCACTATAGG-3' (SEQ ID NO: 4)) was replaced by 5'-TAATACGACTCACTATA-3' (SEQ ID NO: 6). As with the case of (1), ppp-shDIS was synthesized with a T7 RNA polymerase (AmpliScribe™ T7-Flash™ Transcription Kits, EPICENTRE, Cat. No. ASF3507). ppp-shDIS comprises triphosphoric acid, a sense strand, a loop sequence (5'-UUCAAGAGA-3' (SEQ ID NO: 1)), an antisense strand, and UU in that order from the 5' end thereof.

### (4) Evaluation of the interferon β-inducing capacity of pppGG-shDIS

pppGG-shDIS or ppp-shDIS was introduced into HeLa CD4⁺ cells, followed by examination of the IFN β -inducing capacity.

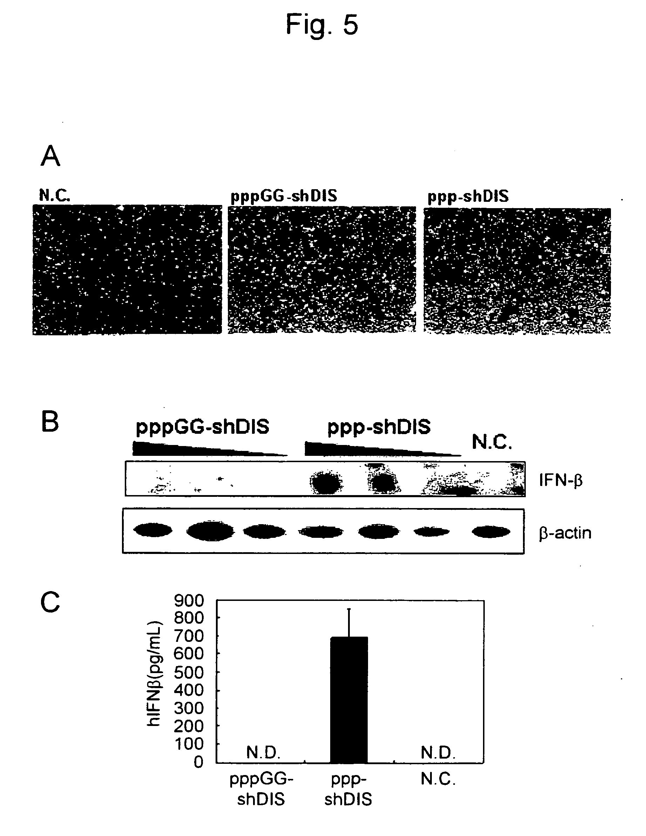

First, HeLa CD4⁺ cells were seeded on a 6-well plate at 1.4 x 10⁵ cells/well, followed by culture in RPMI-1640 (containing 10% FBS, 100 U/ml penicillin, and 100 µg/ml streptomycin) for a day (37°C, 5% CO₂ atmosphere). After culture, the medium was completely removed therefrom and 800 µl of RPMI-1640 (containing 100 U/ml penicillin and 100 µg/ml streptomycin) was freshly added dropwise thereto. DMRIE-C (Invitrogen) and Opti-MEM (Invitrogen) were added to pppGG-shDIS or ppp-shDIS (200, 100, or 50 pmol) (shRNA : reagent = 1 µg : 3 µL), and each resultant was adjusted to 200 µL, followed by incubation at room temperature for 20 minutes. The thus incubated shDIS-reagent complex was added dropwise to HeLa CD4⁺ cells, followed by culture for 1.5 hours. Thus, shDIS was introduced into the cells. After culture, the HeLa CD4⁺ cells were washed with RPMI-1640 (containing 100 U/ml penicillin and 100 µg/ml streptomycin), and 1 mL of RPMI-1640 (containing 10% FBS, 100 U/ml penicillin, and 100 µg/ml streptomycin) was added dropwise thereto, followed by culture for 48 hours. Thereafter, cytopathic effects (CPEs) were microscopically observed (fig. 5A).

In addition, pppGG-shRNA or pppGG-shDIS was introduced in the manner described above, followed by culture for 12 hours. After culture, cells were recovered, followed by analysis of intracellular IFN β expression by the Western blotting method (fig. 5B). Simultaneously, the culture supernatant was recovered, followed by analysis of IFN β expression by the ELISA method (fig. 5C). In the figure, the symbol "N.C." represents a sample into which nothing had been introduced.

As shown in fig. 5A, CPEs were observed by changing the number of bases of the 5' overhang such that CPEs were observed to a greater extent in the case of ppp-shDIS compared with the case of pppGG-shDIS. In addition, as shown in figs. 5B and 5C, as a result of evaluation of the IFN inducing-capacity, IFN-β was induced in the case of ppp-shDIS while IFN-β was not induced in the case of pppGG-shDIS. The same results were obtained in both intracellular (B) and extracellular (C) environments. These results suggested that IFN induction caused by shRNA would possibly depend on the number of bases in the 5' overhang thereof.

### Example 2 Examination of the 5' overhang

### (1) Synthesis of shLuc2

The above results suggested that the IFN-inducing capacity of shRNA synthesized with a T7 RNA polymerase would possibly depend on the number of bases in the 5' overhang thereof. Thus, shRNA having the number of bases in the 5' overhang of 0, 1, 2, or 3 was synthesized. Then, the possibility of such dependency was examined. Further, in order to examine the dependency of shRNA on a target sequence, the target sequence derived from HIV-1 was replaced by a sequence derived from firefly luciferase (shLuc2). Fig. 6 shows the target sequence and 4 types of shLuc2 base sequences.

These 4 types of shLuc2 were synthesized with a T7 RNA polymerase (AmpliScribe™ T7-Flash™ Transcription Kits, EPICENTRE, Cat. No. ASF3507) in the manner described in (1). In addition, the 5' overhang was regulated by changing the T7 promoter base sequence. The respective base sequences of T7 promoters were ppp-shLuc2 (5'-TAATACGACTCACTATA-3' (SEQ ID NO: 6)), pppG-shLuc2 (5'-TAATACGACTCACTATAG-3' (SEQ ID NO: 7)), pppGG-shLuc2 (5'-TAATACGACTCACTATAGG-3' (SEQ ID NO: 4)), and pppGGG-shLuc2 (5'-TAATACGACTCACTATAGGG-3' (SEQ ID NO: 8)).

### (2) Evaluation of interferon β-inducing capacity of shLuc2

shLuc2 was introduced into HeLa CD4⁺ cells, followed by examination of interferon β (IFN β)-inducing capacity.

First, HeLa CD4⁺ cells were seeded on a 6-well plate at 1.4 x 10⁵ cells/well, followed by culture in RPMI-1640 (containing 10% FBS, 100 U/ml penicillin, and 100 µg/ml streptomycin) for a day (37°C, 5% CO₂ atmosphere). After culture, the medium was completely removed therefrom and 800 µl of RPMI-1640 (containing 100 U/ml penicillin and 100 µg/ml streptomycin) was freshly added dropwise thereto. DMRIE-C (Invitrogen) and Opti-MEM (Invitrogen) were separately added to 4 types of shLuc2 (200 pmol) (shRNA : reagent = 1 µg : 3 µL) and each resultant was adjusted to 200 µL, followed by incubation at room temperature for 20 minutes. The thus incubated shLuc2-reagent complex was added dropwise to HeLa CD4⁺ cells, followed by culture for 1.5 hours. Thus, shLuc2 was introduced into the cells. After culture, the HeLa CD4⁺ cells were washed with RPMI-1640 (containing 100 U/ml penicillin and 100 µg/ml streptomycin), and 1 mL of RPMI-1640 (containing 10% FBS, 100 U/ml penicillin, and 100 µg/ml streptomycin) was added dropwise thereto, followed by culture for 12 hours. After culture, cells were recovered, followed by analysis of intracellular IFN β expression by the Western blotting method (fig. 7).

As shown in fig. 7, the 5' overhang-dependent IFN induction was examined. As a result, it was confirmed that IFN tends to be induced inversely to the number of bases in the 5'overhang. In addition, it was confirmed that IFN induction does not depend on a target sequence.

In view of Examples 1 and 2, it was indicated that IFN induction caused by shRNA synthesized with a T7 RNA polymerase is regulated by the 5' overhang of shRNA. In addition, it was suggested that differences in the 5' overhang do not influence PKR/2-5OAS.

### Example 3 Examination of anti-HIV-1 effects of ppp-GGshDIS

pppGG-shDIS (DIS: functional noncoding sequence in HIV genome) or control RNA (LacZ) targeting LacZ was introduced into HeLa CD4⁺ cells. An HIV-1-expressing plasmid (pNL4-3) was introduced thereinto, followed by examination of anti-HIV-1 effects.

First, HeLa CD4⁺ cells were seeded on a 12-well plate at 5 x 10⁴ cells/well, followed by culture in RPMI-1640 (containing 10% FBS, 100 U/ml penicillin, and 100 µg/ml streptomycin) for a day (37°C, 5% CO₂ atmosphere). After culture, the medium was completely removed therefrom and 400 µl of RPMI-1640 (containing 100 U/ml penicillin and 100 µg/ml streptomycin) was freshly added dropwise thereto. DMRIE-C (Invitrogen) and Opti-MEM (Invitrogen) were added dropwise to pppGG-shDIS or LacZ (25, 12.5, or 6.25 pmol) (shRNA : reagent = 1 µg : 3 µL), and each resultant was adjusted to 100 µL, followed by incubation at room temperature for 20 minutes. The thus incubated shDIS-reagent complex was added dropwise to HeLa CD4⁺ cells, followed by culture for 1.5 hours. Thus, shDIS was introduced into the cells. After culture, the HeLa CD4⁺ cells were washed with RPMI-1640 (containing 100 U/ml penicillin and 100 µg/ml streptomycin), and 1 mL of RPMI-1640 (containing 10% FBS, 100 U/ml penicillin, and 100 µg/ml streptomycin) was added dropwise thereto. Fugen^{™}6 (Roche) (DNA : reagent = 1 µg : 3 µL) and Opti-MEM (Invitrogen) were added thereto, followed by incubation at room temperature for 5 minutes. Furthermore, pNL4-3 (100 ng) was added thereto, and each resultant was adjusted to 50 µl, followed by incubation at room temperature for 15 minutes. The thus incubated pNL4-3-reagent complex was added dropwise to HeLa CD4⁺ cells, followed by culture for 48 hours. Thereafter, the culture supernatant was recovered, followed by measurement of the p24 antigen level (fig. 8). As shown in fig. 8, in terms of HIV-1-inhibiting effects, ppp-GGshDIS achieved an approximately 70 % decrease compared with P.C. In addition, decrease in the p24 level was not confirmed upon introduction of control RNA (LacZ). Thus, it was suggested that the above HIV-1-inhibiting effects are not antiviral effects resulting from cytotoxicity caused by shRNA introduction.

### Example 4 Examination of shLuc6

### (1) Synthesis of shLuc6

The results of Example 2 suggested that the IFN-inducing capacity of shRNA synthesized with a T7 RNA polymerase would possibly depend on the number of bases in the 5' overhang thereof. Thus, shRNA having the number of bases in the 5' overhang of 0, 1, 2, or 3 was synthesized. Then, the possibility of such dependency was examined. Further, in order to examine the dependency of shRNA on a target sequence, the target sequence derived from HIV-1 was replaced by a sequence derived from firefly luciferase (shLuc6). A target sequence in a case in which firefly luciferase is a target is shown below.
Luc : 5'-GGAGCCUUCAGGAUUACAAGA-3' (SEQ ID NO: 9)

These 4 types of shLuc6 were synthesized with a T7 RNA polymerase (AmpliScribe™ T7-Flash^{™} Transcription Kits, EPICENTRE, Cat. No. ASF3507) in the manner described in Example 2. In addition, the 5' overhang was regulated by changing the T7 promoter base sequence. The respective base sequences of T7 promoter were ppp-shLuc6 (5'-TAATACGACTCACTATA-3' (SEQ ID NO: 10)), pppG-shLuc6 (5'-TAATACGACTCACTATAG-3' (SEQ ID NO: 11)), pppGG-shLuc6 (5'-TAATACGACTCACTATAGG-3' (SEQ ID NO: 12)), and pppGGG-shLuc6 (5'-TAATACGACTCACTATAGGG-3' (SEQ ID NO: 13)). Fig. 9 shows the structure of shRNA used for evaluation of the IFN-inducing capacity. shRNAs having the number of bases in the 5' overhang of 0, 1, 2, and 3 were constructed. Further, in order to identify factors inducing IFN production, shLuc6 subjected to a dephosphorylation treatment with the use of CIP (Alkaline Phostase, Calf Intestinal, New England Biolabs, Cat. No. 188114) was constructed (fig. 10).

### (2) Evaluation of interferon β-inducing capacity of shLuc6

shLuc6 was introduced into HeLa CD4⁺ cells, followed by examination of interferon β (IFN-β)-inducing capacity.

First, HeLa CD4⁺ cells were seeded on a 12-well plate at 1 x 10⁵ cells/well, followed by culture in RPMI-1640 (containing 10% FBS, 100 U/ml penicillin, and 100 µg/ml streptomycin) for a day (37°C, 5% CO₂ atmosphere). After culture, the medium was completely removed therefrom and 400 µl of RPMI-1640 (containing 100 U/ml penicillin and 100 µg/ml streptomycin) was freshly added dropwise thereto. DMRIE-C (Invitrogen) and Opti-MEM (Invitrogen) were separately added to 4 types of shLuc6 (100 pmol) (shRNA : reagent = 1 µg : 3 µL) and each resultant was adjusted to 200 µL, followed by incubation at room temperature for 20 minutes. The thus incubated shLuc6-reagent complex was added dropwise to HeLa CD4⁺ cells, followed by culture for 1.5 hours. Thus, shLuc6 was introduced into the cells. After culture, the HeLa CD4⁺ cells were washed with RPMI-1640 (containing 100 U/ml penicillin and 100 µg/ml streptomycin), and 1 mL of RPMI-1640 (containing 10% FBS, 100 U/ml penicillin, and 100 µg/ml streptomycin) was added dropwise thereto, followed by culture for 12 hours. After culture, intracellular IFN-β expression was analyzed by the Western blotting method. Fig. 11 shows results of Western blotting for examination of the IFN-β-inducing capacity of shLuc6. The results were obtained by introducing shLuc6 (100 pmol) into HeLa CD4⁺ cells, recovering intracellular proteins 12 hours after introduction, and detecting IFN-β by Western blotting.

In addition, IFN-β expression in the culture supernatant was analyzed by ELISA. Fig. 12 shows results of ELISA for examination of the IFN-β-inducing capacity of shLuc6. The results were obtained by introducing shLuc6 (100 pmol) into HeLa CD4⁺ cells, recovering the culture supernatant 12 hours after introduction, and detecting IFN-β by ELISA. The symbol "N.C." represents a sample into which nothing had been introduced.

In the case of shRNA having a 5' end to which two or more G residues had been added, IFN-β production was not confirmed. These results correlated with the results in the case in which DIS was a target. Thus, even in a case in which a different target sequence was used, IFN production was avoided. Therefore, shRNA can be expected to be useful. Further, since IFN production was avoided by the CIP treatment, it was suggested that an IFN-inducing factor in this study would be triphosphoric acid.

### Industrial Applicability

As shown in Examples, when shRNA comprises a sense strand in which the 5' overhang has one or a plurality of G(s), interferon expression is not induced in cells into which such shRNA has been introduced. Thus, when the shRNA of the present invention is used as a reagent for RNAi research, inhibition of nonspecific gene expression or cytotoxicity caused by an interferon reaction does not take place. Therefore, it is possible to accurately examine inhibition of the expression of a gene or a noncoding region (noncoding sequence) resulting from sequence-specific RNA interference caused by shRNA. Further, when the shRNA of the present invention is used as an RNAi medicine, adverse effects resulting from nonspecific gene expression or cytotoxicity caused by an interferon reaction are not induced *in vivo* so that the shRNA can be used as a safe and effective medicine.
Free Text of Sequence Listing
SEQ ID NOS: 1 to 8: Synthesis

## Claims

1. A short hairpin RNA (shRNA) comprising a sense strand comprising a sequence homologous to a target sequence of target mRNA and an antisense strand comprising a sequence complementary to the sequence of the sense strand and having an overhang consisting of two or three G (guanine) base(s) at the 5' end of the sense strand and triphosphoric acid (ppp) is ligated to the 5' side of the overhang, such shRNA cleaving target mRNA in a sequence-specific manner without causing interferon and/or cytotoxicity induction.

2. The shRNA according to claim 1, wherein the sense strand and the antisense strand have base lengths of 15 to 50.

3. A vector containing template DNA of the shRNA according to claim 1 or 2 and expressing the shRNA, wherein the vector comprises a T7 promoter.

4. A gene expression inhibitor that inhibits *in vitro* the expression of a target gene or a noncoding region comprising a target sequence in a cell without interferon and/or cytotoxicity induction, such inhibitor comprising the shRNA according to claim 1 or 2.

5. A method of inhibiting *in vitro* the expression of a target gene or a noncoding region comprising a target sequence in a cell without interferon and/or cytotoxicity induction, comprising introducing the shRNA according to claim 1 or 2 into a cell.

6. A pharmaceutical composition that does not cause interferon and/or cytotoxicity induction so as to prevent and/or treat a disease involving a gene or a noncoding region comprising a target sequence, such pharmaceutical composition comprising, as an active ingredient, the shRNA according to claim 1 or 2.

7. The pharmaceutical composition according to claim 6, wherein the target sequence is a gene sequence or a noncoding region of a virus and the disease is a viral infectious disease.

8. The pharmaceutical composition according to claim 7 wherein the target sequence is a gene sequence or a noncoding region of HIV.

## Patentansprüche

1. Kurzhaarnadel-RNA (shRNA), umfassend einen Sense-Strang, der eine zu einer Zielsequenz von Ziel-mRNA homologe Sequenz umfasst, und einen Antisense-Strang, der eine zu der Sequenz des Sense-Stranges komplementäre Sequenz umfasst und einen Überhang aufweist, der aus zwei oder drei G- (Guanin-) Basen am 5'-Ende des Sense-Stranges besteht, wobei Triphosphorsäure (ppp) an die 5'-Seite des Überhangs ligiert ist, wobei die shRNA die Ziel-mRNA auf sequenzspezifische Weise spaltet, ohne dabei die Auslösung von Interferon und/oder Zytotoxizität zu verursachen.

2. shRNA nach Anspruch 1, worin der Sense-Strang und der Antisense-Strang Basenlängen von 15 bis 50 aufweisen.

3. Vektor, der Matrizen-DNA von shRNA nach Anspruch 1 oder 2 enthält und die shRNA exprimiert, worin der Vektor einen T7-Promotor umfasst.

4. Genexpressionsinhibitor, der *in vitro* die Expression eines Zielgens oder einer nicht kodierenden Region, die eine Zielsequenz umfasst, in einer Zelle ohne Auslösung von Interferon und/oder Zytotoxizität hemmt, wobei der Inhibitor shRNA nach Anspruch 1 oder 2 umfasst.

5. Verfahren zur Hemmung der Expression eines Zielgens oder einer nicht kodierenden Region, die eine Zielsequenz umfasst, *in vitro* in einer Zelle ohne Auslösung von Interferon und/oder Zytotoxizität, wobei das Verfahren das Einführen von shRNA nach Anspruch 1 oder 2 in eine Zelle umfasst.

6. Pharmazeutische Zusammensetzung, die nicht die Auslösung von Interferon und/oder Zytotoxizität verursacht, um eine Krankheit zu unterbinden und/oder zu behandeln, an der ein Gen oder eine nicht kodierende Region, die eine Zielsequenz umfasst, beteiligt ist, wobei die pharmazeutische Zusammensetzung als aktiven Bestandteil shRNA nach Anspruch 1 oder 2 umfasst.

7. Pharmazeutische Zusammensetzung nach Anspruch 6, worin die Zielsequenz eine Gensequenz oder eine nicht kodierende Region eines Virus ist und die Krankheit eine Vireninfektionskrankheit ist.

8. Pharmazeutische Zusammensetzung nach Anspruch 7, worin die Zielsequenz eine Gensequenz oder eine nicht kodierende Region von HIV ist.

## Revendications

1. ARN en épingle à cheveux (ARNsh) comprenant un brin sens comprenant une séquence homologue à une séquence cible de ARNm et un brin anti-sens comprenant une séquence complémentaire à la séquence du brin sens et ayant un surplomb consistant en deux ou trois bases de G (guanine) à l'extrémité 5' du brin sens, et de l'acide triphosphorique (ppp) est ligaturé au côté 5' du surplomb, un tel ARNsh clivant le ARNm cible d'une manière spécifique à la séquence sans entraîner une induction d'interféron et/ou de cytotoxicité.

2. ARNsh selon la revendication 1, dans lequel le brin sens et le brin anti-sens ont des longueurs de base de 15 à 50.

3. Vecteur contenant l'ADN matrice du ARNsh selon la revendication 1 ou 2 et exprimant le ARNsh, où le vecteur comprend un promoteur T7.

4. Inhibiteur d'expression de gènes qui inhibe in vitro l'expression d'un gène cible ou d'une région de non codage comprenant une séquence cible dans une cellule sans induction d'interféron et/ou de cytotoxicité, un tel inhibiteur comprenant le ARNsh selon la revendication 1 ou 2.

5. Méthode pour inhiber *in vitro* l'expression d'un gène cible ou d'une région de non codage comprenant une séquence cible dans une cellule sans induction d'interféron et/ou de cytotoxicité, comprenant l'introduction du ARNsh selon la revendication 1 ou 2 dans une cellule.

6. Composition pharmaceutique qui n'entraîne pas d'induction d'interféron et/ou de cytotoxicité de manière à prévenir et/ou traiter une maladie impliquant un gène ou une région de non codage comprenant une séquence cible, une telle composition pharmaceutique comprenant, comme ingrédient actif, le ARNsh selon la revendication 1 ou 2.

7. Composition pharmaceutique selon la revendication 6, dans laquelle la séquence cible est une séquence de gènes ou une région de non codage d'un virus, et la maladie est une maladie infectieuse virale.

8. Composition pharmaceutique selon la revendication 7, dans laquelle la séquence cible est une séquence de gènes ou une région de non codage du VIH.


### SEQUENCE LISTING

<110> HaploPharma Inc.
<120> RNAi drug having no side effects
<130> PH-2700-PCT
<150> JP 2005-021960
   <151> 2005-01-28
<160> 13
<170> PatentIn Ver. 2. 1
<210> 1
   <211> 9
   <212> RNA
   <213> Artificial Sequence
<220>
   <223> Description of Artificial Sequence: Synthetic
<400> 1
   uucaagaga 9
<210> 2
   <211> 6
   <212> DNA
   <213> Artificial Sequence
<220>
   <223> Description of Artificial Sequence: Synthetic
<400> 2
   tttttt 6
<210> 3
   <211> 21
   <212> RNA
   <213> Artificial Sequence
<220>
   <223> Description of Artificial Sequence: Synthetic
<400> 3
   ggcuugcuga agcgcgcacg g 21
<210> 4
   <211> 19
   <212> DNA
   <213> Artificial Sequence
<220>
   <223> Description of Artificial Sequence: Synthetic
<400> 4
   taatacgact cactatagg 19
<210> 5
   <211> 55
   <212> RNA
   <213> Artificial Sequence
<220>
   <223> Description of Artificial Sequence: Synthetic
<400> 5
   ggggcuugcu gaagcgcgca cgguucaaga gaccgugcgc gcuucagcaa gccuu 55
<210> 6
   <211> 17
   <212> DNA
   <213> Artificial Sequence
<220>
   <223> Description of Artificial Sequence: Synthetic
<400> 6
   taatacgact cactata 17
<210> 7
   <211> 18
   <212> DNA
   <213> Artificial Sequence
<220>
   <223> Description of Artificial Sequence: Synthetic
<400> 7
   taatacgact cactatag 18
<210> 8
   <211> 20
   <212> DNA
   <213> Artificial Sequence
<220>
   <223> Description of Artificial Sequence: Synthetic
<400> 8
   taatacgact cactataggg 20
<210> 9
   <211> 21
   <212> RNA
   <213> Artificial Sequence
<220>
   <223> Description of Artificial Sequence: Synthetic
<400> 9
   ggagccuuca ggauuacaag a 21
<210> 10
   <211> 17
   <212> DNA
   <213> Artificial Sequence
<220>
   <223> Description of Artificial Sequence: Synthetic
<400> 10
   taatacgact cactata 17
<210> 11
   <211> 18
   <212> DNA
   <213> Artificial Sequence
<220>
   <223> Description of Artificial Sequence: Synthetic
<400> 11
   taatacgact cactatag 18
<210> 12
   <211> 19
   <212> DNA
   <213> Artificial Sequence
<220>
   <223> Description of Artificial Sequence: Synthetic
<400> 12
   taatacgact cactatagg 19
<210> 13
   <211> 20
   <212> DNA
   <213> Artificial Sequence
<220>
   <223> Description of Artificial Sequence: Synthetic
<400> 13
   taatacgact cactataggg 20